# EUROPEAN PATENT SPECIFICATION

(11) **EP 1 673 492 B1**
(45) Date of publication and mention of the grant of the patent: **18.03.2009**
(21) Application number: 04765955.2
(22) Date of filing: 13.10.2004
(51) Int. Cl.: C25D 3/54, H05H 6/00

(54) **RADIUM TARGET AND METHOD FOR PRODUCING IT**
RADIUM-TARGET UND HERSTELLUNGSVERFAHREN DAFÜR
CIBLE DE RADIUM ET METHODE DE PRODUCTION DE LA CIBLE

(30) Priority: 13.10.2003 DE 10347459
(43) Date of publication of application: 28.06.2006
(73) Proprietor: Actinium Pharmaceuticals Inc., Florham Park, NJ 07932 (US)
(72) Inventor: MORENO BERMUDEZ, Josue, Manuel, 85737 Ismaning (DE); TÜRLER, Andreas, 85737 Ismaning (DE); HENKELMANN, Richard, 85354 Freising (DE); HARFENSTELLER, Mark, 80796 München (DE); HÜENGES, Ernst, 85748 Garching (DE); SCHILP, Michael, 85748 Garching (DE); BUCK, Oliver, 83457 Bayerisch Gmain (DE); GEERLINGS, Mauritius, W., 83440 Montaeuroux, Var (FR)
(74) Representative: Winter, Brandl, Fürniss, Hübner Röss, Kaiser, Polte Partnerschaft Patent- und Rechtsanwaltskanzlei
(86) International application number: PCT/EP2004/011510
(87) International publication number: WO 2005/039647

(56) References cited:
- EP-A- 0 962 942
- J. OF RADIOANALYTICAL AND NUCLEAR CHEMISTRY, vol. 160, no. 2, 1992, pages 477-485, XP008047777 cited in the application
- INGELBRECHT C ET AL: "Improved electrodeposited actinide layers" NUCLEAR INSTRUMENTS & METHODS IN PHYSICS RESEARCH, SECTION - A: ACCELERATORS, SPECTROMETERS, DETECTORS AND ASSOCIATED EQUIPMENT, NORTH-HOLLAND PUBLISHING COMPANY. AMSTERDAM, NL, vol. 397, no. 1, 21 September 1997 (1997-09-21), pages 34-38, XP004093028 ISSN: 0168-9002

## Description

The present invention refers to a method for producing a radium target for the production of radionuclides by means of accelerated protons according to the preamble of claim 1. Further, the invention refers to a radium target according to claim 25.

In particular, the targets according to the present invention serve for the production of radionuclide ²²⁵Ac, which is successfully used in nuclear medicine - bound to tumorspecific antibodies - in various clinical trials in the treatment of cancer, particularly in form of its daughter nuclide ²¹³Bi.

Already in 1993, criteria for the selection of radionuclides for immunotherapy with α-emitters and β-emitters were provided for the first time by GEERLINGS (GEERLINGS, M.W. (1993): Int. J. Biol. Markers, 8, 180-186: "Radionuclides for radioimmunotherapy: criteria for selection") where it turned out due to the difference in energy that the radioactivity of α-emitters to be applied may be more than 1000 times lower than that of β-emitters, if a comparable effect is to be achieved.

Moreover, in the work of GEERLINGS 1993 the α-emitting radionuclides ²²⁵Ac und its daughter isotope ²¹³Bi turned out to be highly promising for the objects of radioimmunotherapy alongside the in principle usable, however relatively poorly available or instable antibody conjugate producing α-emitters: ²¹¹At, ²⁵⁵ Fm, ²¹² Bi/²¹² Pb, ²²⁴Ra, ²³³ Ra.

One of the fundamental studies for the foundation of a radioimmunotherapy with α-emitters is disclosed in GEERLINGS, M.W., KASPERSEN, F.M., APOSTOLIDIS; C. and VAN DER HOUT, R. (1993): Nuclear Medicine Communications 14, 121-125, "The feasibility of 225Ac as a source of α-particles in radioimmunotherapy". Here it is described that ²²⁵ Ac produced from ²²⁹Th and the daughter isotope of ²²⁵Ac**,** namely ²¹³Bi is suitable as isotope for the radioimmunotherapy with α-emitters. As indications there are described in particular cancer treatment and the treatment of micrometastases of malign tumors using tumor-specific monoclonal antibodies as carriers for α-emitters.

A further study of KASPERSEN, F.M., BOS,E., DOORNMALEN, A.V., GEERLINGS, M.W., APOSTOLIDIS, C. and MOLINET, R. (1995): Nuclear Medicine Communications, 16, 468-476: "Cytotoxicity of 213Bi - and 225Ac - immunoconjugates" confirms and quantifies the cytotoxic effect of ²¹³Bi and ²²⁵Ac with in vitro tests using the human epidermoid tumor cell line A431.

Moreover, it is suggested to use ²¹³Bi for the treatment of malignant diseases of the blood system.

Further, in KASPERSEN et al. 1995 a process can be found with which antibodies can be bound chemically to a chelator suitable for ²¹³Bi and ²²⁵Ac. It has proved that for example p-isothiocyanatobenzyl-diethylentriamine-pentaacetate (benzyl-DTPA) is particularly suitable.

Another chelator, namely Cyclohexyl-DTPA is, for example, described in NIKULA, T.K., McDEVITT, M.R., FINN, R.D., WU, C., KOZAK, R.W., GARMESTANI, K., BRECHBIEL, M.W., CURCIO, M.J., PIPPIN, C.G., TIFFANY-JONES, L., GEERLINGS, M.W.,Sr., APOSTOLIDIS, C., MOLINET, R., GEERLINGS, M.W.,Jr., GANSOW, O.A. UND SCHEINBERG, D.A. (1999): J Nucl Med, 40, 166-176: "Alpha-Emitting Bismuth Cyclohexylbenzyl DTPA Constructs of Recombinant Humanized Anti-CD33 Antibodies: Pharmacokinetics, Bioactivity, Toxicity and Chemistry".

An overview over chelator chemistry can be found for example in HASSFJELL, S. und BRECHBIEL, W. (2001): Chem. Rev., 101, 2019-2036: "The Development of the α-Particle Emitting Radionuclides 212 Bi and 213Bi, and Their Decay Chain Related Radionuclides, For Therapeutic Applications"

In the meantime, various radioimmunotherapeutic approaches with ²²⁵_{Ac} and ²¹³Bi for the treatment of cancer are in various phases of clinical trials.

The medical-clinical significance of the present invention may be seen for example from two promising therapeutic approaches:
On the one hand, JURCIC, J.G., LARSON, S.M., SGOUROS, G., McDEVITT, M.R., FINN, R.D., DIVGI, C.R. se, M.B:, HAMACHER, K.A:, DANGSHE, M., HUMM, J.L., BRECHBIEL, M.W., MOLINET, R., SCHEINBERG, D.A. (2002) in Blood, 100, 1233-1239 report a significant success in the treatment of patients with acute myelogenous leukaemia (AML) and chronic myelogenous leukaemia (CML) by using ²¹³Bi, which is bound to HuM195, a formulation of a monoclonal anti-CD33-antibody, which was developed for the humane medicine. This study was the first proof-of concept where a human being was treated with a systemic radioimmunotherapy comprising an α-emitter, which is transported to a tumorspecific cellular target.
On the other hand, HUBER, R., SEIDL, C., SCHMID, E, SEIDENSCHWANG, S., BECKER; K.-F., SCHUMACHER; C., APOSTOLIDIS, C., NIKULA, T., KREMMER, E., SCHWAIGER, M. and SENEKOWITSCH-SCHMIDTKE, R. (2003): Clinical Cancer Research (Suppl.) 9, 1s-6s: "Locoregional α-Radioimmunotherapy of Intraperitoneal Tumor Cell Dissemination Using a Tumor-specific Monoclonal Antibody" report the therapeutic effectivity of ²¹³Bi-d9MAB - with low bone marrow toxicity - and the possible application of a locoregional therapy for patients who suffer from gastric carcinoma, who express d9-E-Cadherine.

More results of studies and partial aspects in this matter are shown in: Roswitha HUBER, doctorate dissertation in the Faculty of Veterinary Medicine submitted to the Ludwig-Maximilians-University of Munich, July 18, 2003: "Bewertung der lokoregionalen Radioimmuntherapie disseminierter Tumorzellen des diffusen Magenkarzinoms mit einem ²¹³Bi gekoppelten tumorspezifischen Antikörper im Mausmodell" (Evaluation of a locoregional radioimmunotherapy of disseminated tumor cells of the diffuse gastric carcinoma with a ²¹³Bi bound tumor specific antibody in the mouse model).

This dissertation was originated from Nuklearmedizinische Klinik and Poliklinik of the Technical University of Munich, the University hospital "Klinikum rechts der Isar", dean: Prof. Dr. M. Schwaiger. The dissertation was prepared under the supervision of Prof. Dr. med. Dr. phil. Reingard Senekowitsch-Schmidtke and was presented to the veterinary faculty via Prof. Dr. med. vet. K. Tempel; Institute for Pharmacology, Toxicology and Pharmacy of the Faculty of Veterenary Medicine of the Ludwig-Maximilians-University of Munich, director: Prof. Dr. med. vet. R. Schulz.

According to HUBER 2003, each year 18 out of 100 000 Germans come down with gastric carcinoma alone. In Japan, even 126 out of 100 000 people are affected. This means about 156 000 incidences per year in Japan alone. There, as well as in China, Taiwan and Korea, gastric carcinoma is one of the most frequent causes of death in consequence of a tumor. When a peritoneal carcinomatosis, the consequence of diffuse expansion of tumor cells in the abdominal cavity, is diagnosed, the life expectancy of a patient is at present about 12 months. Even with resectable gastric carcinoma, this means with carcinoma, which have not yet disseminated and with negative diagnostic findings with respect to lymph nodes, the relapse-free three-year survival-rate is at about 45%, only.

Up to now the application of cytostatica within a chemotherapy seemed to be the most promising therapeutic way.

However, the side effects range from immunosuppression, coagulopathy, metabolic anoxia, mucositis and hyperuricaemia to the danger of cytostatica induced secondary tumors. Particularly affected is here quickly proliferating tissue as bone marrow and the epithelium of the gastrointestinal tract as well as of the oral mucosa.

The radioimmunotherapy, in contrast, uses protein structures located on the membrane, that are expressed by tumor cell lines in order to bind cytotoxic active substances via a carrier. Mostly, an overexpression of the binding molecule at the tumor cell is central to a radioimmunotherapy. The target molecule for the tumor associated antibodies is thus also expressed to a lower extend in physiologic cells of the organism. This implies that any therapeutic agent for radiotherapy also binds to these cells.

Particularly, in the treatment of acute or chronic myelogenous leukaemia the meaning of the present invention takes effect, namely for the preparation of a suitable α-emitter, namely ²²⁵Ac which forms through decay reaction the bound, for example, to a tumorspecific antibody.

The ²¹³Bi atom decays via ß-decay to ²¹³Po, which releases its α-decay energy of 8,4 MeV with a half life of 4 µs in the tissue within a distance of 80µm when decaying and thus kills effectively cells in its immediate neighborhood due to its high linear energy transfer.

The so called locoregional application enables a quick binding of ²¹³Bi bound tumor specific antibody to the tumor antigenes with maximal therapeutic success and minimal toxicity.

Not before the late 80s was the α-emitting nuclide pair ²¹³Bi/²¹³Po was discovered for radioimmunotherapy and further examined by GEERLlNGS 1993. However, in the standard textbook of Schicha and Schober, 1997 "Nuklearmedizin - Basiswissen und klinische Anwendung" (nuclear medicine - basic knowledge and clinical application) it can be read: *"The linear energy transfer of α-rays is* so *big that the likeliness for the creation of irradiation damages is bigger than a therapeutic effect. For this reason, nuclides, which release α-rays, are not applied in the nuclear medicine...". ("Der lineare Energietransfer ist bei α-Strahlen so groß, daß die Wahrscheinlichkeit for die Erzeugung von Strahlenschäden großer ist als ein therapeutischer Effekt. Aus diesem Grunde werden, Nuklide, die α-Strahlen emittieren, in der Nuklearmedizin...nicht eingesetzt.*")

However, in the clinical application of such α-emitters in combination with tumorspecific antibodies, exactly the opposite has proved to be true (cf. JURCIC et al. 2002). Consequently, the question arose which isotope it was best to use and how it could be prepared reliably and continuously.

Most of the over hundred available α-emitters can already be excluded from *in vivo* application for practical reasons (cf. GEERLINGS 1993). These α-emitters have to meet requirements like sufficient chemical and physical purity, economic availability and an adequate half-life. The latter has to be long enough for binding to the antibodies and for the biologic allocation and has to be short enough so that the patient is not put at an unnecessary risk due to excessive exposition to the rays.

One of the few α-emitter which fulfil these criteria (cf. GEERLINGS 1993) is the nuclide pair ²¹³Bi/²¹³Po with a half-life of 45,6 min (²¹³Bi). The photon emission of ²¹³Bi with 440 KeV additionally permits an in vivo scintiscanning of the patient as well as an easy measurement of the activity using an α-ray counter.

Moreover, in radiation protection it is important that the radiation can be detected easily. Furthermore, also traces of further daughter nuclides of ²²⁵Ac/ ²¹³Bi as for example 221 Fr or ²⁰⁹Pb can be determined by new methods of measurement and can also be included into the dosimetry alongside the quality control.

In the meantime, ²¹³Bi has become available via the production of ²²⁵Ac, for example according to EP 0 752 709 B1 and EP 0 962 942 A1 and particularly via the so called "thorium cow" according to US 5,355,394. However, the production via the above-mentioned "thorium cow" is very expensive, as it derives from a neutron irradiation of ²²⁶Ra over several years, whereby finally among others an isotope mixture of ²²⁸Th and ²²⁹Th is assembled, whereby ²²⁹Th again decays via ²²⁵Ra into ²²⁵Ac, which decays to ²¹³ Bi.

Thus, the mother-daughter nuclide pair ²²⁵Ac /²¹³Bi is available in principle, however, neither in an adequate quantity and continously nor at an acceptable price, however - as mentioned initially - first clinical studies with ²²⁵Ac/²¹³Bi conjugated to HuM195, a humanized anti-CD33 monoclonal antibody are very successful against myeloid leukaemia. The first clinical phase I trials with ²¹³Bi -HuM195 were carried out with excellent therapeutic results at leukaemia patients at the Memorial Sloan-Kettering Cancer Center in New York (JURICIC et al. 2002).

In cyclotrons, developed for the first time 1931, electrically charged particles are moving on spiral shaped orbits in magnetic flux lines.

In particular, protons can be accelerated with the help of a cyclotron with currents that are high enough to such high velocities that they can be used in experimental and applied nuclear physics for the production of isotopes in a quantitative scale.

EP 0 752 709 B1 describes, for example, a method for producing Actinium-225 from Radium-226, whereby accelerated protons are projected in a cyclotron onto a target of radium-226, characterized in that protons accelerated in a cyclotron are projected onto a target of radium-226 in a cyclotron, so that the instable compound nucleus ²²⁷Ac is transformed into Actinium-225 while emitting two neutrons (p,2n-reaction), whereby after a waiting period, during which the Actinium-226, which has been created simultaneously due to the emission of only one neutron, decays mostly due to its considerably shorter half-life and Actinium is chemically separated, so that an almost exclusively pure isotope Ac-225 is obtained.

The ²²⁶Ra target used according to the procedure of EP 0 752 709 B1 is not specified in detail there.

EP 0 962 942 A1 also describes a method for producing Ac-225 by irradiation of ²²⁶Ra with protons, which are accelerated in a cyclotron to an energy of 10 to 20 MeV.

According to the prior art of EP 0 962 942 A1, the target nuclide ²²⁶Ra is used in the form of RaCl₂, which can be obtained for example by precipitation with concentrated HCl or radiumcarbonate (RaCO₃). These radium substances are then pressed into target pellets. Prior to irradiation of the radium salts with protons, the pellets are heated to about 150°C in order to release crystal water and are then sealed in a silver capsule. The capsule is then mounted on a frame-like support and connected to a water cooling circuit. The target itself exhibits a window, which is arranged in a way that the proton beam hits the target through the window. According to EP 0 962 942 A1, the target exhibits a surface of about 1 cm².

Although it is already possible to achieve good Actinium-225-yields with the targets according to EP 0 962 942 A1, it has turned out in practice that this target construction can heat itself under certain conditions due to the proton beam in such a way that the silver capsule tears open and might thus both destroy the target and contaminate the peripheral compounds.

As a result, it is the object of the present invention to provide improved radium targets for the production of radionuclides by means of accelerated protons, on the basis of the prior art of EP 0 962 942 A1.

With respect to a method, the above object is achieved by the characterising features of claim 1.

With regard to a radium target, the above object is achieved by the *characterising features of claim 25.*

Central to the present invention is a process for producing a radium target for the production of radio nuclides by means of accelerated protons, wherein an electrodeposition of radium containing material of at least one aqueous-organic solution, which contains radium ions, is carried out on at least one aluminium surface, whereby the aluminium surface is connected as cathode.

Though it is known in principle from Haïssinsky, M.J., Chim. Phys. 34, 321 (1937) "Electrolyse de sels de baryum et de radium dans l'acétone" to electrodeposit radium from barium/radium mixtures from acetone in thin films on cathodes made of platinum, gold, silver, nickel or copper, an application as target for the transformation of radionuclides in a proton beam of an accelerator, like a cyclotron or a linear accelerator, is not mentioned.

Besides, N.E. Whitehead, R.G. Ditchbum, W.J. McCabe, R. Van der Raaij, describe in J. of Radioanalytical and Nuclear Chemistry, Articles, Vol. 160, No. 2 (1992) 477-485 "Factors affecting the electrodeposition of RA-226" an electrolytic deposition of ²²⁶Ra out of "90% isopropyl alcohol or ethyl alcohol in an acidic environment at 35 V and an with an electric current of 100mA over a time period of 20 minutes on stainless steel discs to carry out a α-spectroscopy.

Targets as defined by the present invention are also not mentioned there.

According to the present invention it is preferred to use a solution of a ²²⁶Ra - salt, in particular nitrate, as these salts are particularly well soluble in aqueous-alcoholic solutions, for example in 70 to 90 % isopropanol.

However, ²²⁶Ra chlorides or ²²⁶Ra carbonates can also be used, which are transformed for the electrodeposition, preferably before the carrying out of the electrodeposition, by means of HNO₃ into the nitrate salt.

According to the present invention, it is preferred to prepare the radionuclide ²²⁵Ac from ²²⁶Ra by means of cyclotron accelerated protons or by means of linear accelerated protons, as with the targets of the present invention it becomes possible for the first time to produce Actinium-225 continuously for the production of radioimmunotherapeutic compounds as for example ²²⁵Ac - and ²¹³Bi labelled antibodies, in particular monoclonal antibodies, for the radioimmunotherapy of cancer and metastases.

These radioimmunochemical methods are for example summarised nuclear chemically and clinically in the dissertation of HUBER, München 2003, which was mentioned in the introduction.

The radiotherapeutic effect essentially takes place through the daughter isotopes of Actinium-225, namely Bismuth-213 and the Polonium-213 resulting therefrom, which is particularly suitable as α-emitter for highly specific and locally restricted irradiation of tumors.

The electrodeposition of ²²⁶Ra material out of the aqueous-organic solution preferably takes place in an acidic environment, whereby nitric acid is used as mineral acid.

In this context it has turned out that an 0.05 molar solution of nitric acid is particularly suitable in order to positively influence the electrodeposition of ²²⁶Ra containing material.

It has proved advantageous to select the alcohol out of the following group consisting of: linear and branched C₁-C₅ alkyl alcohols; ethanol, propanol-1, propanol-2, acetone as well as mixtures thereof.

The advantage of these organic solvents lies in the fact that the radium salts are particularly well soluble therein. It has proved further that as a rule a concentration of an organic solvent in water of 70 to 90% leads to the best results.

Further it is advantageous to add ammonium ions to the aqueous-organic solution of the ²²⁶Ra salts, as after the deposition of the radium containing material the film of radium oxides/hydroxides and/or peroxides formed on the aluminium surface is stabilised, or fixed, respectively, by ammonium ions.

According to the present invention the use of an aluminium foil, which exhibits for the purposes of the present invention a purity of at least 99% and for example a thickness of about 0.01 mm to 0.05 mm, in particular preferred about 0.015 mm, as aluminium surface is preferred. The advantage lies in the fact that the aluminium foil is industrially available in various sizes and thicknesses and thus it can be made use of as a base material that is readily available and furthermore relatively cheap.

Due to the corrosion protection and/or the fact that it is inert, it has turned out at the implementation of the present method according to the invention that a platin anode as counter electrode yields the best results for the electrodeposition of radium.

The method according to the invention is carried out preferably with a D.C. voltage of about 10 to 600 V, in particular about 200V and an electric current of about 20 to 1000 mA, in particular about 60 mA, and at a pH value of about 4 to 5 or about 11, since at this value the most even layers of ²²⁶Ra material on the aluminium surface are achieved.

It is a preferred embodiment of the present invention to arrange the aluminium foil for the carrying out of the electrodeposition of ²²⁶Ra on a support, whereby a support made of stainless steel is particularly preferred. The advantage lies in the fact that the aluminium foil can easily be connected as cathode over the conductive stainless steel support.

Of course, it is also possible to use an electrically inert support, for example made of plastics, whereby the aluminium foil is connected via a connected electrode as cathode.

According to the invention, it is preferred to rotate the support during the electrodeposition, as by doing so an even coating with the desired radium isotope, especially at bigger coating thicknesses, is achieved.

On the one hand, through these measures, a basically circular-shaped aluminium disc with radium containing material can be coated largely on the whole surface.

On the other hand it is also possible to coat only the outer edge of the circular-shaped disc in a ring shaped manner with radium containing material.

In this process, support and aluminium foil partially dip into an aqueous-organic solution containing radium ions, and support as well as aluminium foil rotate during the electrodeposition, so that a ring shaped coating with radium containing material is obtained.

As due to the size of the irradiation window only a small coating width is required for the target itself, the ring shaped coating is sufficient and thus combines the advantages of an easy and safe carrying out of the method and obtains at the same time an optimum yield for the proton nuclear reaction.

In order to increase the yield of the proton irradiation, the obtained, aluminium disc, which is largely coated on the whole surface with radium, is folded repeatedly for the creation of the target used in the proton beam.

This easy measure enables an increase of yield with the given target geometry of the irradiation window.

The method currently preferred to build the target used in the proton beam is, however, to pile up a plurality of the obtained discs which are coated with radium in a ring shaped manner, also in order to increase the effective cross section of the proton radiation.

In various studies it has turned out that the carrying out of the method according to the invention with target discs piled up this way yields the best results with regard to ²²⁵Ac yield and with regard to contamination security in the use of the coated aluminium discs.

It is an alternative method to unwind the aluminium foil of a supply coil in a galvanic cell containing the aqueous-organic solution with radium ions and to guide it between two anodes;
to subject it to the electrodeposition of radium for a pre-determined period of time in order to deposit radium containing layers on both aluminium surfaces; and
to wind up the radium-coated aluminium foil to a coil.

Preferably, the aluminium coil is wound up under pressure with a roll.

In this embodiment of the present invention, the high surface density of the deposited ²²³Ra -containing material that was obtained due to the two-sided coating is only to be achieved by a relatively high procedural effort, compared to the piled up aluminium foils.

It is advantageous to fix the deposited ²²⁶Ra -containing films on the aluminium foil, as due to this measurement they adhere particularly tight and with a large abrasion resistance to the aluminium surface. The preferred fixing agent is NH₃, which may be added to the plating solution about one minute before the termination of the electrodeposition.

For further improvement, radium containing films on the aluminium foil are dewatered, in particular by IR irradiation. This has the advantage that for the nuclear transformation by means of accelerate protons the target virtually does no longer contain water and thus, the danger of steam creation, which may be produce undesired pressures in the target capsule and may severely disturb the whole target system by creating cracks in the layer, can largely be avoided.

It is of great advantage if the method according to the invention can be carried out continuously, since thereby in an industrial or semi industrial process a bigger amount of ²²⁶Ra targets can be produced for the continuous production of radioimmuno antibodies for therapeutic purposes and can be stored at least for a short period of time.

Furthermore, it is preferred to carry out the whole procedure in an inert gas atmosphere. This way an unfavourable influence on the deposition process by oxygen-caused oxidation processes is avoided.

If required, the aluminium foil used for the electrodeposition of radium-containing material may additionally be surface activated by the usual measures.

The radium targets obtained by the method according to the invention may then be subjected to proton irradiation with sufficient energy in a cyclotron or in a linear accelerator, for example between about 10 and 25 MeV, more preferably between about 18 and 23 MeV, in order to obtain the desired ²²⁵Ac.

For the production of radionuclides themselves, it is referred to the teaching of EP 0 752 709 B1 and EP 0 962 942 A1 and which are incorporated by reference.

An ²²⁵Ac thus obtained is bound for example to antibodies for radioimmunotherapy. Such procedures of coupling are well known to those skilled in the art and can be found for example in KASPERSEN et al. 1995 as well as in HUBER, 2003.

The typical radium targets according to the present invention have the form of aluminium foil, which at least contains on one surface a layer made of radium containing material, particularly radium oxide and/or radium peroxide and/or radium hydroxide.

A preferred embodiment of the present invention is a radium target, in which the radium-coated aluminium foil is present in folded form, as wound coil or as pile of single foils coated with radium containing material.

Therein, the radium content of the radium containing layer may lie within the nanogram range to gram range in form of the radium oxide and/or peroxide and/or hydroxide.

Particularly preferred for the purposes of the present invention is a radium target, which exhibits an activity of about 1 *n*Ci to 1.5 Ci, preferably 500 *m*Ci of ²²⁶Ra.

Further, it is preferred to form a circular disc shaped radium target, whereby it is present as circular disc shaped radium coated aluminium foil which exhibits the radium coating preferably formed in a ring shaped manner on the outer edge of the aluminium circular disc.

A particularly preferred radium target of the present invention is one where it is present as pile of several ring shaped radium-coated circular discs made of aluminium.

Alternatively, the radium target may be present in a folded form, particularly several times folded, if the aluminium foil is largely coated on the whole surface with radium containing material.

Another possibility of the target form is to form it as rectangular formed foil und to wind it into a coil. Thereby it is possible to store a relatively big amount of target foil and separate required pieces of foil like in the use of an "aluminium foil for the household".

On the other hand, it is also possible to use the wound coil - if the dimensioning is adapted to the conditions of the accelerator - the foil itself as target.

Alternatively, Al-mesh targets can be used as carrier of Ra.

Al-mesh targets have an advantage in the achieved yield during electrodeposition. With the introduction of the Al-mesh disc as cathode in the electrodeposition process and as carrier of Ra in the target, the amount of Ra that can be deposited per disc could be increased. While, e.g. on an Al-foil disc the amount of Ra (experiments conducted at mg levels with Ba and at microgram levels with Ra-226) deposited was below 10 mg (2-3 mm at the edges of one disc), in the case of the mesh disc, the amount of Ra was to approximately 70 mg (depending on the thickness of the deposit and other parameters, thicker deposits were not well adhered to the mesh anymore). Consequently the number of Ra/Al mesh discs that need to be introduced into the target cup was reduced to five or six instead of 10 or more as it was required by the use of Al-foil discs. The better yield of electrodeposition on Al mesh compared with the yield of Al foil is associated with the higher surface of the mesh. The fact that more Ra is electrodeposited on the Al also assures that the proton beam is hitting with higher probability the Ra and not much loss occurs in Al.

The dimensions of the Al-mesh might be for example:

| | |
|---|---|
| Nominal spacing: | appr. 0,11 mm |
| Wire diameter: | appr. 0,1 mm |
| Total open area: | 27 mm² |

The improvement by using an Al-mesh also facilitated the automation of the process.

Preferably, a 99 % pure Al provided by Good Fellow is used. The neutron activation results carried out on the mesh at the Institute are reported below:
Impurities in the Al mesh measured by ko-INAA are given in Table 1

**Table 1**

| Element | Content [µg/g] | Element | Content [µg/g] |
|---|---|---|---|
| Fe | 1302 | La | 0.69 |
| Cr | 701 | W | 0.2 |
| Ni | 0.2 | Sb | 0.07 |
| Ga | 145 | Th | 0.18 |
| Zn | 39 | Br | 0.11 |
| Na | 9 | Sm | 0.08 |
| Mo | 3.5 | As | 0.06 |
| U | 1.3 | Sc | 0.02 |
| Co | 2.0 | Au | 0.002 |
| Ce | 1.8 | | |

As in the case of the Al-foil targets, the results from processing hundreds microCi of Ra/Al-mesh discs targets indicated that the selective leaching of Ra and Ac from the Al mesh (developed for the Al disc target) can be also performed. Already during the dissolution of the target is possible to separate most of the Al and impurities from the Ac.

A special advantage of the radium targets according to the invention is that they exhibit basically pure radium material in their radium containing coating. Hereby it is achieved that the targets are free of carriers or diluents, for example barium salts, which had to be added to the conventional radium targets of the prior art, i.e. the target pellets mentioned in the introduction, in order to homogenize the radium-containing material. Due to the possibility to be able to work without such carrier materials as barium compounds, the chemical separation and purification of the created ²²⁵Ac becomes substantially more simple and the yields of irradiation are optimized, as competitive nuclear reactions, as for example those from barium nuclei, are not possible.

Further advantages and features can be seen from the description of the examples.

### Example 1 - deposition by means of a fixed aluminium disc as cathode

For the preparation of a ²²⁶Ra target, aluminium discs with a thickness of 0.015mm and a diameter of about 5 cm with a minimal 99% purity of the aluminium are punched out and fixed on a stainless steel support. The support facilitates the handling of the aluminium foils and is removed after the electrodeposition itself, before the positioning of the radium-coated foil in the target itself.

For the electrodeposition of the aluminium foil, a solution of a radium-226-nitrate is used, whereby in particular 226-radium chloride or 226-radium carbonate are absorbed beforehand for the transformation into the corresponding nitrate in about 0.05 M HNO₃.

Subsequently, the stainless steel support, on which the aluminium foil is fixed, is weighted and the net weight of the aluminium foil is determined.

150ml (for electrodeposition on aluminium foils with a diameter of up to 15 cm) or 10 to 11 ml isopropanol are added into an electrodeposition cell (for aluminium foil discs with a diameter up to 2cm).

Then the required amount of radium-226 solution is filled into the electrolytic cell and 1-2ml 0.05 M HNO₃ are added. The total volume of the radium solution and 0.05 M HNO₃ should not exceed about 2 ml, if aluminium foil discs with a diameter of up to 2 cm are used, and 20 ml at the most, if aluminium foil discs with a diameter of up to 15 cm are used. When high radium concentrations are used, a white precipitates may be formed. If this happens, 0.05 MHNO₃ is further added until the precipitation has dissolved. The pH value of the depositing plating solution should preferably be between 4 and 5.

For the electrodeposition of ²²⁶Ra containing material out of the plating solution the electric current is adjusted to about 60 mA and a voltage of about 200V is applied, monitored for a few minutes and, if necessary, readjusted.

### Example 2 - deposition by means of a rotating aluminium disc as cathode

In a preferred embodiment, the stainless steel support with the aluminium foil fixed on it is, however, being dipped about 5 mm into the electroplating solution according to example 1 and a platin anode (Pt-conductor or Pt-net) is arranged within a distance of about 1 cm of the aluminium/stainless steel cathode and the stainless steel carrier is rotated with the aluminium foil arranged on it by means of a motor drive. For the electrodeposition of ²²⁶Ra containing material out of the plating solution the electric current is adjusted to about 60 mA and a voltage of about 200V is applied, monitored for a few minutes and, if necessary, readjusted.

Furthermore, the dipping depth of the aluminium disc to be coated, or the level of the solution, respectively, are kept at a constant level during the coating period.

Subsequently the deposition takes place for about 20-30 minutes at 60 mA. A decrease of the voltage after 20 to 30 minutes indicates the termination of the electrodeposition.

When the voltage does not change any more in time, about 0.5 or 1 ml of an ammonia solution are added to the cell and after a waiting period of one minute, the obtained radium-containing film is fixed. Normally, a quantitative electrodeposition might time from about 20 to 40 minutes the deposition on aluminium foils with a diameter of up to 2 cm, while a deposition on aluminium foils with up to 15 cm diameter might time about 2 to 3 hours. The Al-target discs prepared in the example with a diameter of about 5.5 cm take about 1 hour for the radium deposition.

After the electrodeposition of the ²²⁶Ra solution has been completed, the plating solution is poured out, the support is rinsed with 2 to 3 ml isopropanol and the cell is disassembled and the aluminium foil is additionally rinsed with about 1 to 2 ml isopropanol.

Afterwards, the support with the ²²⁶Ra coated aluminium foil arranged on it is dried under an infrared lamp until the weight remains constant, in order to render the radium-containing coating anhydrous.

Afterwards, the stainless steel support with the fixed, coated aluminium foil is weighted and the net mass of the coated aluminium foil is determined. Then the yield is determined from the weighted mass of the ²²⁶Ra containing layer.

An alternative way to monitor the yield of the electrodeposition - instead of weightening - is to measure the γ-activity of ²²⁶Ra by means of a high resolution γ-spectrometer.

Subsequently, the stainless steel support and the aluminium foil are separated from each other.

The dry aluminium foil coated with radium compounds is carefully covered with a new aluminium foil and the edges of the aluminium foil with which the Aluminium foil carrying the active layer is fixed are cut off, in order to minimize the amount of aluminium in the target itself.

For the use as radium target in the proton beam of a cyclotron, a pile of the of the circular disc shaped aluminium foils prepare according to present example 15, which are coated with radium-containing material in a ring shaped manner, are piled in a so called target cup.

For the production of a folded radium target, one or more aluminium foils, in the case of this example, coated on one whole surface with ²²⁶Ra are covered in a way with another aluminium foil that the radium containing film is covered entirely. Then, the aluminium foil is folded several times until stripes of about 2mm are obtained. The folded aluminium foil, which contains the layers of radium-containing material, in particular radium oxides, is then placed into the target for proton irradiation in the cyclotron or in the linear accelerator,

With the method according to the present invention, it is possible to obtain highly potent ²²⁶Ra targets on aluminium foil of a different thickness with different ²²⁶Ra-amounts.

The method according to the present invention permits in particular to deposit films that are highly homogenous on the aluminium- ²²⁶Ra target. This is particularly important for the irradiation of the target in the cyclotron, as the atomic nuclei of radium are thereby exposed homogenously to the proton flux.

The use of aluminium as substrate for ²²⁶Ra offers various advantages for the irradiation in a cyclotron and the subsequent radiochemical processing of the irradiated target. The advantages of the aluminium lie in the nuclear physical and chemical properties of the aluminium:
Nuclear properties: Aluminium has just one single stable isotope. The activation products formed from the aluminium are very short-lived. The formation of only short lived radionuclides on aluminium facilitates the radiochemical purification of Ac-225 and reduces the cooling time of the target after irradiation. As the loss of energy of protons in aluminium is very low, it is possible to use several thin films of aluminium without substantial reduction of the proton energy.
Physical properties: Aluminium is a light metal with good thermal and electrical conductivity. It is easy to handle and can be adapted easily to the required geometry.
Chemical properties: Aluminium can easily be dissolved in mineral acids and it can be easily separated from the resulting Actinium. Aluminium foils are available with a high degree of chemical purity and at reasonable prices.

The deposition of ²²⁶Ra as oxide or peroxide allows to obtain a layer with a high content of radium, in particular higher than 70% of the deposited material per cm². The electrodeposition yield is high if all the instructions of the present invention are followed.

In practice it has turned out that about 43 to 5 g/cm ²²⁶Ra with good adhesive properties can be deposited on the aluminium foil.

The method facilitates the eventual automation of the target production process. This aspect is very important for the radiation safety and the continuity of the process.

The use of folded aluminium layers as substrate for ²²⁶Ra facilitates the sample processing, as after the irradiation these foils can be easily removed from the target supports without loosing their mechanical integrity. This prevents the loss of material and the radioactive contamination of the compounding line, which otherwise could not be prevented.

## Claims

1. Method for producing a radium target for the production of radionuclide by means of accelerated protons,
**characterized in that**
an electrodeposition of radium containing material out of at least one aqueous-organic solution containing radium ions is carried out on at least one aluminium surface, wherein the aluminium surface is connected as cathode.

2. Method according to claim 1, **characterized in that** a solution of one ²²⁶Ra salt, in particular nitrate, is used.

3. Method according to claim 1 or 2, **characterized in that** the protons are accelerated by means of a cyclotron or a linear accelerator.

4. Method according to claims 1 to 3, **characterized in that** the radionuclide ²²⁵Ac is produced from ²²⁶Ra by means of protons accelerated in a cyclotron.

5. Method according to any one of claims 1 to 4, **characterized in that** the aqueous-organic solution contains at least one mineral acid and at least one alcohol.

6. Method according to claim 5 **characterized in that** nitric acid is used as mineral acid.

7. Method according to claim 6, **characterized in that** nitric acid is used as 0.05 molar solution.

8. Method according to any one of claims 5 to 7, **characterized in that** the alcohol is selected from the group consisting of: linear and branched C₁- C₅ alkyl alcohols; ethanol, propanol-1, propanol-2; acetone as well as mixtures thereof.

9. Method according to any one of claims 1 to 8, **characterized in that** the aqueous-organic solution contains ammonium ions.

10. Method according to any one of claims 1 to 9, **characterized in that** as aluminium surface an aluminium foil or mesh is used.

11. Method according to claim 10, **characterized in that** the aluminium foil or mesh exhibits a degree of purity of at least 99% and a thickness of 0.01 mm to 0.05 mm, in particular about 0.015 mm.

12. Method according to any one of claims 1 to 11, **characterized in that** at least one platinum anode is used as counter electrode for the electrodeposition of radium.

13. Method according to any one of claims 1 to 12, **characterized in that** the electrodeposition is carried out with a D.C. voltage of 10 to 600 V, in particular about 200V and a current of 20 to 1000 mA, in particular of about 60 mA and at a pH value of about 4 to 5.

14. Method according to any one of claims 1 to 13, **characterized in that** the aluminium foil for carrying out of the electrodeposition of radium is arranged on a support, in particular one made of stainless steel.

15. Method according any one of claims 1 to 14, **characterized in that** the support rotates during the electrodeposition.

16. Method according to any one of claims 1 to 15, **characterized in that** an obtained aluminium disc being preferably largely coated with radium on the whole surface, is folded for the creation of the target used in the proton beam, in order to increase the ²²⁵Ac yield.

17. Method according to any one of claims 1 to 15, **characterized in that** a circular shaped aluminium disc is coated in a ring shaped manner on the outer edge of the circular shaped disc, wherein support and aluminium foil or mesh are partially dipped into an aqueous-organic solution containing radium ions, and support as well as aluminium foil are rotating during the electrodeposition, so that a ring shaped coating with radium-containing material is achieved.

18. Method according to claim 17, **characterized in that** for the formation of the target used in the proton beam, a plurality of obtained aluminium discs, which are coated in a ring shaped manner with radium, is piled, in order to increase the ²²⁵Ac yield.

19. Method according to any one of claims 1 to 13, **characterized in that** the aluminium foil is unwound from a storage coil into a galvanic cell containing the aqueous-organic solution with radium ions, and is directed between two anodes;
is subjected to the radium electrodeposition process for a pre-determined period of time in order to deposit radium containing layers on both aluminium surfaces; and
the radium-coated aluminium foil or mesh is wound up to a coil.

20. Method according to claim 19, **characterized in that that** the aluminium coil is wound under pressure with a roll.

21. Method according to any one of claims 1 to 20, **characterized in that** the radium containing layers are fixed on the aluminium foil or mesh, in particular by means of NH₃.

22. Method according to any one of claims 1 to 21, **characterized in that** the radium containing films on the aluminium foil or mesh are dried, in particular by means of IR irradiation.

23. Method according to any one of claims 1 to 22, **characterized in that** it is carried out continuously.

24. Method according to any one of claims 1 to 23, **characterized in that** it is carried out in an inert gas atmosphere.

25. Radium target, **characterized in that** it is obtained by a method according to at least one of claims 1 to 24.

26. Radium target according to claim 25, **characterized in that** it contains an aluminium foil which contains at least on a part of the surface a layer of radium containing material, in particular radium oxide and/or radium peroxide and/or radium hydroxide.

27. Radium target according to claim 25 or 26, **characterized in that** the radium-coated aluminium foil is present in folded form, as wound coil or as pile of single foils or meshes coated with radium containing material.

28. Radium target according to any one of claims 25 to 27, **characterized in that** it contains radium within the nanogram range to gram range in form of the radium oxide and/or peroxide and/or hydroxide.

29. Radium target according to one of claims 25 to 28, **characterized in that** it exhibits an activity of about 1 nCi to 1.5 Ci, preferably 500 mCi of ²²⁸Ra.

30. Radium target according to one of claims 25 to 29, **characterized in that** it is present as circular disc shaped radium coated aluminium foil or mesh which exhibits the radium coating preferably formed in a ring shaped manner on the outer edge of the aluminium circular disc.

31. Radium target according to claim 30, **characterized in that** it is present as a pile of single radium coated circular shaped discs made of aluminium, wherein they are preferably coated in a ring shaped manner at the outer edge.

32. Radium target according to any one of claims 25 to 31, **characterized in that** it is present in folded form, wherein the aluminium foil or mesh preferably is coated largely on the whole surface with radium containing material.

33. Radium target according to claim 27, **characterized in that** it is present in rectangular form, in particular as wound up coil.

34. Radium target according to any one of claims 25 to 33, **characterized in that** the radium salt on the aluminium surface is largely free of carrier material as, for example, barium salt.

## Patentansprüche

1. Verfahren zur Herstellung eines Radium-Targets für die Radionuklidherstellung mittels beschleunigter Protonen,
**dadurch gekennzeichnet, dass**
eine elektrolytische Abscheidung von Radium-haltigem Material aus wenigstens einer wässrig-organischen Lösung, die Radiumionen enthält, auf wenigstens einer Aluminiumoberfläche durchgeführt wird, wobei die Aluminiumoberfläche als Kathode geschaltet wird.

2. Verfahren nach Anspruch 1, **dadurch gekennzeichnet, dass** eine Lösung eines ²²⁶Ra-Salzes, insbesondere Nitrat, verwendet wird.

3. Verfahren nach Anspruch 1 oder 2, **dadurch gekennzeichnet, dass** die Protonen mittels eines Zyklotrons oder eines Linearbeschleunigers beschleunigt werden.

4. Verfahren nach den Ansprüchen 1 bis 3, **dadurch gekennzeichnet, dass** das Radionuklid ²²⁵Ac aus ²²⁶Ra mittels zyklotronbeschleunigten Protonen hergestellt wird.

5. Verfahren nach einem der Ansprüche 1 bis 4, **dadurch gekennzeichnet, dass** die wässrig-organische Lösung wenigstens eine Mineralsäure und wenigstens einen Alkohol enthält.

6. Verfahren nach Anspruch 5, **dadurch gekennzeichnet, dass** Salpetersäure als Mineralsäure verwendet wird.

7. Verfahren nach Anspruch 6, **dadurch gekennzeichnet, dass** die Salpetersäure als 0,05 molare Lösung verwendet wird.

8. Verfahren nach einem der Ansprüche 5 bis 7, **dadurch gekennzeichnet, dass** der Alkohol ausgewählt wird aus der Gruppe bestehend aus: linearen und verzweigten C₁-C₅-Alkylalkoholen; Ethanol, Propanol-1, Propanol-2; Aceton, sowie Mischungen daraus.

9. Verfahren nach einem der Ansprüche 1 bis 8, **dadurch gekennzeichnet; dass** die wässrig-organische Lösung Ammoniumionen enthält.

10. Verfahren nach einem der Ansprüche 1 bis 9, **dadurch gekennzeichnet, dass** als Aluminiumoberfläche eine Aluminiumfolie oder ein Aluminiumgitter verwendet wird.

11. Verfahren nach Anspruch 10, **dadurch gekennzeichnet, dass** die Aluminiumfolie oder das Aluminiumgitter einen Reinheitsgrad von wenigstens 99% und eine Dicke von 0,01 mm bis 0,05 mm, insbesondere ungefähr 0,015 mm, aufweist.

12. Verfahren nach einem der Ansprüche 1 bis 11, **dadurch gekennzeichnet, dass** zur elektrolytischen Abscheidung von Radium wenigstens eine Platinanode als Gegenelektrode verwendet wird.

13. Verfahren nach einem der Ansprüche 1 bis 12, **dadurch gekennzeichnet, dass** die elektrolytische Abscheidung mit einer Gleichspannung von 10 bis 600 V, insbesondere ungefähr 200 V und einem Strom von 20 bis 1000 mA, insbesondere ungefähr 60 mA und bei einem pH-Wert von ungefähr 4 bis 5 durchgeführt wird.

14. Verfahren nach einem der Ansprüche 1 bis 13, **dadurch gekennzeichnet, dass** die Aluminiumfolie für die Durchführung der elektrolytischen Abscheidung von Radium auf einem Träger, insbesondere einem solchen aus Edelstahl, angeordnet wird.

15. Verfahren nach einem der Ansprüche 1 bis 14, **dadurch gekennzeichnet, dass** der Träger während der elektrolytischen Abscheidung rotiert.

16. Verfahren nach einem der Ansprüche 1 bis 15, **dadurch gekennzeichnet, dass** eine erhaltene, vorzugsweise im Wesentlichen vollflächig Radium-beschichtete Aluminiumscheibe zur Bildung des im Protonenstrahl verwendeten Targets mehrfach gefaltet wird, um die ²²⁵Ac-Ausbeute zu erhöhen.

17. Verfahren nach einem der Ansprüche 1 bis 15, **dadurch gekennzeichnet, dass** eine kreisscheibenförmige Aluminiumscheibe am Außenrand der Kreisscheibe ringförmig beschichtet wird, wobei Träger und Aluminiumfolie oder Aluminiumgitter in eine wässrig-organische Lösung, die Radiumionen enthält, teilweise eintauchen, und Träger sowie Aluminiumfolie während der elektrolytischen Abscheidung rotieren, so dass eine ringförmige Beschichtung mit Radium-haltigen Material erhalten wird.

18. Verfahren nach Anspruch 17, **dadurch gekennzeichnet, dass** zur Bildung des im Protonenstrahl verwendeten Targets eine Mehrzahl von erhaltenen, ringförmig Radium-beschichteten Aluminiumscheiben gestapelt wird, um die ²²⁵Ac-Ausbeute zu erhöhen.

19. Verfahren nach einem der Ansprüche 1 bis 13, **dadurch gekennzeichnet, dass** die Aluminiumfolie von einer Vorratsspule in eine galvanische Zelle, die die wässrig-organische Lösung mit Radiumionen enthält, abgespult und zwischen zwei Anoden geführt wird;
für eine bestimmte Zeit dem elektrolytischen Radium-Abscheideprozess ausgesetzt wird, um auf beiden Aluminiumoberflächen Radium-haltige Schichten abzuscheiden; und
die Radium-beschichtete Aluminiumfolie oder das Radium-beschichtete Aluminiumgitter zu einer Spule aufgewickelt wird.

20. Verfahren nach Anspruch 19, **dadurch gekennzeichnet, dass** die Aluminiumspule mit einer Walze unter Druck aufgewickelt wird.

21. Verfahren nach einem der Ansprüche 1 bis 20, **dadurch gekennzeichnet, dass** die Radium-haltigen Schichten auf der Aluminiumfolie oder dem Aluminiumgitter fixiert werden, insbesondere mittels NH₃.

22. Verfahren nach einem der Ansprüche 1 bis 21, **dadurch gekennzeichnet, dass** die Radium-haltigen Filme auf der Aluminiumfolie oder auf dem Aluminiumgitter getrocknet werden, insbesondere mittels IR-Bestrahlung.

23. Verfahren nach einem der Ansprüche 1 bis 22, **dadurch gekennzeichnet, dass** es kontinuierlich durchgeführt wird.

24. Verfahren nach einem der Ansprüche 1 bis 23, **dadurch gekennzeichnet, dass** es in einer Inertgasatmosphäre durchgeführt wird.

25. Radium-Target, **dadurch gekennzeichnet, dass** es nach einem Verfahren gemäß wenigstens einem der Ansprüche 1 bis 24 erhalten wird.

26. Radium-Target nach Anspruch 25, **dadurch gekennzeichnet, dass** es eine Aluminiumfolie enthält, die auf wenigstens einem Teil der Oberfläche eine Schicht aus Radium-haltigem Material, insbesondere Radiumoxid und/oder Radiumperoxid und/oder Radiumhydroxid, enthält.

27. Radium-Target nach Anspruch 25 oder 26, **dadurch gekennzeichnet, dass** die Radium-beschichtete Aluminiumfolie in gefalteter Form, als aufgewickelte Spule oder als Stapel von mit Radium-haltigem Material beschichteten Einzelfolien oder Einzelgittern vorliegt.

28. Radium-Target nach einem der Ansprüche 25 bis 27, **dadurch gekennzeichnet, dass** es Radium vom Nanogrammbereich bis zum Grammbereich als Radiumoxid und/oder Peroxid und/oder Hydroxid enthält.

29. Radium-Target nach einem der Ansprüche 25 bis 28, **dadurch gekennzeichnet, dass** es eine Aktivität von ungefähr 1 nCi bis 1,5 nCi, vorzugsweise 500 mCi, an ²²⁶Ra aufweist.

30. Radium-Target nach einem der Ansprüche 25 bis 29, **dadurch gekennzeichnet, dass** es als kreisscheibenförmige Radium-beschichtete Aluminiumfolie oder Aluminiumgitter vorliegt, wobei die Radium-Beschichtung vorzugsweise ringförmig am äußeren Rand der Aluminiumkreisscheibe vorliegt.

31. Radium-Target nach Anspruch 30, **dadurch gekennzeichnet**, das es als Stapel von einzelnen Radium-beschichteten Kreisscheiben aus Aluminium vorliegt, wobei sie vorzugsweise ringförmig am äußeren Rand beschichtet sind.

32. Radium-Target nach einem der Ansprüche 25 bis 31, **dadurch gekennzeichnet, dass** es in gefalteter Form vorliegt, wobei die Aluminiumfolie oder das Aluminiumgitter vorzugsweise im Wesentlichen vollflächig mit Radium-haltigem Material beschichtet ist.

33. Radium-Target nach Anspruch 27, **dadurch gekennzeichnet, dass** es in Rechteckform, insbesondere als aufgewickelte Spule, vorliegt.

34. Radium-Target nach einem der Ansprüche 25 bis 33, **dadurch gekennzeichnet, dass** das Radiumsalz auf der Aluminiumoberfläche im Wesentlichen frei von Trägermaterialen, beispielsweise Bariumsalz, ist.

## Revendications

1. Procédé de production d'une cible de radium pour la production de radionucléide au moyen de protons accélérés,
**caractérisé en ce que**
une électrodéposition de matière contenant du radium parmi au moins une solution organique aqueuse contenant des ions radium est effectuée sur au moins une surface d'aluminium, la surface d'aluminium étant connectée en tant que cathode.

2. Procédé selon la revendication 1, **caractérisé en ce qu'**une solution d'un sel ²²⁶Ra, en particulier du nitrate, est utilisée.

3. Procédé selon la revendication 1 ou 2, **caractérisé en ce que** les protons sont accélérés au moyen d'un cyclotron ou d'un accélérateur linéaire.

4. Procédé selon les revendications 1 à 3, **caractérisé en ce que** le radionucléide ²²⁵Ac est produit à partir de ²²⁶Ra au moyen de protons accélérés dans un cyclotron.

5. Procédé selon l'une quelconque des revendications 1 à 4, **caractérisé en ce que** la solution organique aqueuse contient au moins un acide minéral et au moins un alcool.

6. Procédé selon la revendication 5, **caractérisé en ce que** de l'acide nitrique est utilisé en tant qu'acide minéral.

7. Procédé selon la revendication 6, **caractérisé en ce que** de l'acide nitrique est utilisé en tant que solution molaire à 0,05.

8. Procédé selon l'une quelconque des revendications 5 à 7, **caractérisé en ce que** l'alcool est choisi dans le groupe comprenant les alcools d'alkyle en C₁-C₅ linéaires et ramifiés ; l'éthanol, le propanol-1, le propanol-2 ; l'acétone ainsi que des mélanges de ceux-ci.

9. Procédé selon l'une quelconque des revendications 1 à 8, **caractérisé en ce que** la solution organique aqueuse contient des ions ammonium.

10. Procédé selon l'une quelconque des revendications 1 à 9, **caractérisé en ce qu'**en tant que surface d'aluminium, une feuille ou maille d'aluminium est utilisée.

11. Procédé selon la revendication 10, **caractérisé en ce que** la feuille ou maille d'aluminium présente un degré de pureté d'au moins 99 % et une épaisseur de 0,01 mm à 0,05 mm, en particulier, de 0,015 mm environ.

12. Procédé selon l'une quelconque des revendications 1 à 11, **caractérisé en ce qu'**au moins une anode de platine est utilisée en tant que contre-électrode pour l'électroposition de radium.

13. Procédé selon l'une quelconque des revendications 1 à 12, **caractérisé en ce que** l'électrodéposition est effectuée avec une tension C.C. de 10 à 600 V, en particulier, de 200 V environ et un courant de 20 à 1000 mA, en particulier, de 60 mA environ et à une valeur de pH de 4 à 5 environ.

14. Procédé selon l'une quelconque des revendications 1 à 13, **caractérisé en ce que** la feuille d'aluminium pour effectuer l'électrodéposition de radium est agencée sur un support, en particulier, un support en acier inoxydable.

15. Procédé selon l'une quelconque des revendications 1 à 14, **caractérisé en ce que** le support tourne pendant l'électrodéposition.

16. Procédé selon l'une quelconque des revendications 1 à 15, **caractérisé en ce qu'**un disque en aluminium obtenu, de préférence recouvert en grande partie de radium sur l'ensemble de sa surface, est plié pour la création de la cible utilisée dans le faisceau de protons, afin d'augmenter le rendement ²²⁵Ac.

17. Procédé selon l'une quelconque des revendications 1 à 15, **caractérisé en ce qu'**un disque en aluminium de forme similaire est revêtu à la manière d'un anneau sur le bord externe du disque de forme circulaire, dans lequel le support et la feuille ou maille en aluminium sont partiellement dopés dans une solution organique aqueuse contenant des ions radium, et le support ainsi que la feuille d'aluminium tournent pendant l'électrodéposition, de sorte à obtenir un revêtement en forme d'anneau avec une matière contenant du radium.

18. Procédé selon la revendication 17, **caractérisé en ce que** pour la formation de la cible utilisée dans le faisceau de protons, une pluralité de disques en aluminium obtenus, qui sont revêtus à la manière d'un anneau avec du radium, est empilée, afin d'augmenter le rendement ²²⁵Ac.

19. Procédé selon l'une quelconque des revendications 1 à 13, **caractérisé en ce que** la feuille d'aluminium est déroulée à partir d'une bobine de stockage dans une cellule galvanique contenant la solution organique aqueuse avec des ions radium, et est dirigée entre deux anodes ;
est soumise au processus d'électrodéposition de radium pendant une période de temps prédéterminée afin de déposer des couches contenant du radium sur les deux surfaces d'aluminium ; et
la feuille ou la maille d'aluminium revêtue de radium est enroulée sur une bobine.

20. Procédé selon la revendication 19, **caractérisé en ce que** la bobine d'aluminium est enroulée sous pression avec un rouleau.

21. Procédé selon l'une quelconque des revendications 1 à 20, **caractérisé en ce que** les couches contenant du radium sont fixées sur la feuille ou maille d'aluminium, en particulier au moyen de NH₃.

22. Procédé selon l'une quelconque des revendications 1 à 21, **caractérisé en ce que** les films contenant du radium sur la feuille ou maille d'aluminium sont séchés, en particulier, au moyen d'un rayonnement IR.

23. Procédé selon l'une quelconque des revendications 1 à 22, **caractérisé en ce qu'**il est effectué en continu.

24. Procédé selon l'une quelconque des revendications 1 à 23, **caractérisé en ce qu'**il est effectué dans une atmosphère de gaz inerte.

25. Cible de radium, **caractérisée en ce qu'**elle est obtenue par un procédé selon au moins une des revendications 1 à 24.

26. Cible de radium selon la revendication 25, **caractérisée en ce qu'**elle contient une feuille d'aluminium qui contient au moins sur une partie de sa surface une couche de matière contenant du radium, en particulier, de l'oxyde de radium et/ou du péroxyde de radium et/ou de l'hydroxyde de radium.

27. Cible de radium selon la revendication 25 ou 26, **caractérisée en ce que** la feuille d'aluminium revêtue de radium est présente sous forme pliée, sous forme de bobine enroulée ou sous forme de pile de simples feuilles ou mailles revêtues de matière contenant du radium.

28. Cible de radium selon l'une quelconque des revendications 25 à 27, **caractérisée en ce qu'**elle contient du radium dans la plage du nanogramme au gramme sous forme d'oxyde et/ou de péroxyde et/ou d'hydroxyde de radium.

29. Cible de radium selon une des revendications 25 à 28, **caractérisée en ce qu'**elle présente une activité d'environ 1 nCi à 1,5 Ci, de préférence, de 500 mCi de ²²⁶Ra.

30. Cible de radium selon une des revendications 25 à 29, **caractérisée en ce qu'**elle est présente sous la forme d'une feuille ou maille d'aluminium revêtue de radium en forme de disque circulaire qui présente le revêtement de radium de préférence formé à la manière d'un anneau sur le bord extérieur du disque circulaire en aluminium.

31. Cible de radium selon la revendication 30, **caractérisée en ce qu'**elle est présente sous la forme d'une pile de simples disques en aluminium de forme circulaire revêtus de radium, dans laquelle ils sont de préférences revêtus à la manière d'un anneau sur leur bord externe.

32. Cible de radium selon l'une quelconque des revendications 25 à 31, **caractérisée en ce qu'**elle est présente sous forme pliée, dans laquelle la feuille ou maille d'aluminium est de préférence largement revêtue sur l'ensemble de sa surface avec une matière contenant du radium.

33. Cible de radium selon la revendication 27, **caractérisée en ce qu'**elle est présente sous forme rectangulaire, en particulier, comme une bobine enroulée.

34. Cible de radium selon l'une quelconque des revendications 25 à 33, **caractérisée en ce que** le sel de radium sur la surface d'aluminium est en grande partie exempt de matériau porteur, par exemple, de sel de baryum.
